# EUROPEAN PATENT SPECIFICATION

(11) **EP 1 411 889 B1**
(45) Date of publication and mention of the grant of the patent: **03.01.2007**
(21) Application number: 02747792.6
(22) Date of filing: 30.06.2002
(51) Int. Cl.: A61K 8/35, A61K 8/44, A61K 8/49, A61K 31/385, A61K 31/197, A61K 31/122, A61K 45/06, A61Q 19/00

(54) **CREAM FOR TREATMENT OF SKIN INJURED BY THE SUN**
CREME ZUR BEHANDLUNG VON SONNENGESCHÄDIGTER HAUT
CREME DE SOIN POUR LES PEAUX ENDOMMAGEES PAR LE SOLEIL

(30) Priority: 02.07.2001 SE 0102380
(43) Date of publication of application: 28.04.2004
(73) Proprietor: Macronova AB, 851 22 Sundsvall (SE)
(72) Inventor: ANDERSSON, Johan, S-856 41 Sundsvall (SE); STARLANDER, Ulf, S-113 35 Stockholm (SE); ANDERSSON, Björn, S-610 21 Norsholm (SE)
(74) Representative: Akerman, Marten Lennart
(86) International application number: PCT/SE2002/001308
(87) International publication number: WO 2003/037291

(56) References cited:
- EP-A2- 0 945 127
- EP-A2- 1 175 898
- WO-A1-00/11968
- WO-A1-00/53176
- WO-A1-02/09657
- WO-A2-02/09652
- DE-A1- 10 020 874
- DE-A1- 19 806 947
- US-A- 5 912 272
- US-A- 5 977 162
- US-B1- 6 300 377
- DATABASE WPI Week 199840, Derwent Publications Ltd., London, GB; Class B02, AN 1990-287822, XP002956506 & JP 2 204 417 A (MARUZEN KASEI CO LTD) 14 August 1990
- DATABASE CAPLUS [Online] DOC. NO. 131:78144 NEUMANN PETRA ET AL.: 'A new liquid thickener for all kinds of cosmetic emulsions', XP002956507 Retrieved from STN Database accession no. 1999:419578 & SOFW. J. vol. 125, no. 6, 1999, pages 8 - 10

## Description

This invention refers to a cream of the kind described in the preamble of claim 1 for topical treatment of visibly photodamaged skin caused by processes involving the influence of free radicals.

The invention also refers to a vehicle for said cream in accordance with the preamble of claim 4 and a method in producing the cream as described in the preamble of claim 6.

In every age and culture, humans have used different means of improving their looks, and a youthful appearance has become increasingly idealised. A person's personal characteristics are strongly connected to her face. Thus, facial creams and make-up are designed to accentuate and reinforce youthful features and to conceal and diminish the signs of ageing. A youthful appearance may also be accentuated with a tan.

The cells of the skin are injured when exposed to the sunlight and UV-radiation, which eventually causes a visible injury - photodamaged skin - which is characterised by locally exaggerated pigmentation, looseness, fine lines, wrinkles, enlarged pores, bags under the eyes, and the development of black or darkened plugs in the sebaceous glands. The photodamaged skin is aggravated by smoking, inappropriate nutrition, illness and stress.

Nowadays it is commonly accepted that photodamaged skin is caused by short-term intermediates of oxygen and nitrogen known as free radicals, which are produced mostly in the mitochondria of the cell. Free radicals may injure the genetic material of cells and mitochondria and also reduce the energy production of the mitochondria. Since free radicals are commonly occurring, certain defence mechanisms are inherent at cell level, including the cooperation of antioxidative nutrients, reparative enzymes and various adaption mechanisms.

Damage that has not been repaired accumulates, leading to a gradually diminishing energy production and an increase in the production of free radicals.

There have been continuous efforts to affect the skin and to arrest or delay the development of photodamaged skin by the topical application of antioxidative compounds. One example of this is the attenuation of the phototoxic response by using the antioxidant vitamins C and E.

There is also substantial evidence that antioxidants may act as anti-inflammatory agents on a cellular level by inhibiting the activation of certain inflammatory related enzymes (kinases), as well as transcription factors such as nuclear factor κB. The use of antioxidants on the skin may prevent expression of the genes of proinflammatory cytokines such as TNF-α, IL-1, IL-2, IL-6 and IL-8, which are all of importance in the origin of inflammatory responses to sun exposure.

A large number of existing creams contain active ingredients with radical scavenging properties. One such ingredient is retinoic acid, the only ingredient so far where there is a scientific consensus that it in certain respects can have a positive effect on photodamaged skin.

Other creams are disclosed in WO00/53176, which contains a combination of lipoic acid and the amino-acid cystein, EP 0 945 127 which may contain acetylcarnitine in combination with other antioxidants, e.g. lipoic acid, DE 198 06 947 A1 which may contain acetylcarnitine and Q-10, and US 5 912 272 containing Q-10. Furthermore, there are several creams containing antioxidants that jointly contribute to the energy production of the cells. What these creams have in common is that they are purported to have an effect on skin changes related to ageing, which can hardly be said to be unique. None of the creams mentioned above claims synergetic effects between lipoic acid, Q-10 and acetylcarnitine in well-balanced proportions.

The purpose of this invention is to obtain a cream with a capability to diminish, to a considerable degree, the visible signs of photodamaged skin. This is obtained by using a cream made in accordance with this invention, whose characterising properties are described in claim 1.

Like retinoic acid, this cream diminishes the visible signs of photodamaged skin without causing the side effects typical of retinoic acid.

Initially, some people may have a strong reaction to the amount of d,l-α-lipoic acid in a cream of this kind, whereas this invention permits the use of a cream with less of d,1-α-lipoic acid. This cream has the characterising properties mentioned in claim 3.

Another purpose of the invention is to obtain a vehicle which can enclose and stabilise the active ingredients of the cream, and from which they can perform in an optimal way during a sufficiently long period of time. The vehicle has been provided with the characterising properties mentioned in claim 4.

Yet another purpose of the invention is to obtain a method with which to disperse and emulsify d,1-α-lipoic acid in the vehicle when manufacturing the cream. This is obtained by a method with the characterising properties of claim 5.

Lipoic acid is a potent fat- and water-soluble antioxidant, which can be synthesised by plants as well as by animals. The antioxidative activity is present both in its oxidised and in its reduced form, dihydrolipoic acid. Lipoic acid efficiently scavenges, inter alia, the hydroxyl radicals, singlet oxygen and nitric oxide, and acts as a modulator of the inflammatory response within the cell. At least theoretically, the size and the solubility characteristics of the molecule ought to establish better conditions for a clinical effect on skin compared to other acknowledged antioxidants. In a topical application to the skin the lipoic acid will rapidly penetrate the horny layer of the epidermis and can be found in the dermis within approximately 4 hrs. Lipoic acid is easily transformed to its reduced form, and the effects of both lipoic acid and dihydrolipoic acid is apparent on the intra- as well as the extracellular level following an exogenous application of lipoic acid.

Coenzyme Q-10 can be synthesised by plants as well as by animals, as can lipoic acid. The self-synthesis of Q-10 in humans occurs through the mevalonate chain, where several fats and fat-like substances are synthesised. Q-10 acts as an antioxidant in its reduced form, ubiquinol. The concentration of ubiquinol is particularly high in the epidermis contra the dermis compared to other antioxidants. It may be interpreted as a greater need in the epidermis of antioxidant protection through ubiquinol . The assimilation of Q-10 applied to the skin occurs relatively quickly and is determined by the concentration and the duration of contact. When Q-10 is applied to the skin in a solution of ethyl alcohol, and after penetrating the horny layer of the epidermis, about 20 % will reach the living part of epidermis and 2 % the dermis.

Apart from their antioxidative properties, both lipoic acid and Q-10 have a fundamental role in the energy production of the cell. Lipoic acid is present in several enzyme systems in the citric acid cycle of the mitochondrion. It has been shown that a supplement of lipoic acid will improve the performance of the mitochondrion and may reverse some of the processes involved in ageing. Q-10 forms a part of the electron transport chain where the substance will increase the electron transmitting capacity as well as the electric potential over the inner membrane of the mitochondrion.

Another substance included in the cream that is presented in this invention is acetylcarnitine. Acetylcarnitine is included in the cream for its capacity to, in catalytic quantities, restore the fatty acid oxidation of the mitochondrion, which is impaired in aged mitochondria.

In controlled studies, Q-10 to some extent has been shown to have an effect on photodamaged skin. Acetylcarnitine is found in various skin care products, but without any reports of effects on photodamaged skin. Lipoic acid has in an open uncontrolled clinical study been shown to have an effect on photodamaged skin to a certain extent.

Combining lipoic acid with Q-10 and acetylcarnitine in specified amounts produces a remarkable synergetic effect where the combination of all three substances gives an enhanced effect to photodamaged skin compared to having lipoic acid separately and Q-10 and acetylcarnitine separately in one and the same vehicle.

The cream described in the present invention contains a water-based vehicle, which has been specially developed to disperse and emulsify lipoic acid, Q-10 and acetylcarnitine. Furthermore, the components of the vehicle are aimed at giving the cream a suitable consistency, and act as a softening agent as well as a moistening preservation agent. An appropriate preparation to obtain said synergetic effect has the following ingredients: between 0.5 and 7 % by weight d,1-α-lipoic acid, preferably 5.0 % by weight, between 0.05 and 0.5 % by weight coenzyme Q-10, where 0.3 % by weight is a well balanced amount, between 0.01 and 3 % by weight acetyl-1-carnitine hydrochloride, where 0.03 % by weight is a well balanced amount, and these are included in an ideal vehicle constituting a base formula for the above mentioned ingredients, containing water in 20 - 80 % by weight, where 46.32 % by weight is a well balanced weight, 5 - 50 % by weight xanthangum in a 2 % water solution, where 15.0 % by weight is a well balanced amount, 1 - 40 % by weight of caprylic/capric triglyceride, where 13.0 % by weight is a well balanced amount, 1- 25 % by weight of Prunus dulcis oil, where 4.0 % by weight is a well balanced amount, 0.5 - 10 % by weight PEG-75 stearate, where 3.3 % by weight is a well balanced amount, 0.5 - 10 % by weight glyceryl stearate, where 2.7 % by weight is a well balanced amount, 1 - 10 % by weight cetearyl alcohol, where 3.0 % by weight is a well balanced amount, 1 - 10 % by weight dimethicone, where 3.0 % by weight is a well balanced amount, 1 - 15 % by weight glycerin, where 3.0 % by weight is a well balanced amount, 0.1- 1.0 % by weight phenoxyethanol, where 0.355 % by weight is a well balanced amount, 0.05 - 1.0 % by weight methylparaben, where 0.085 % by weight is a well balanced amount, 0.005 - 0.5 % by weight butylparaben, where 0.02 % by weight is a well balanced amount, 0.005 - 0.5 % by weight ethylparaben, where 0.02 % by weight is a well balanced amount, 0.005 - 0.5 % by weight propylparaben, where 0.01 % by weight is a well balanced amount, 0.005 - 0.5 % by weight isobutylparaben, where 0.01 % by weight is a well balanced amount, 0.05 - 5 % by weight sodium hydroxide in 10 % water solution, where 0.4 % by weight is a well balanced amount, 0.05 - 5 % by weight perfume, where 0.25 % by weight is a well balanced amount and 0.05 - 5 % by weight carbomer, where 0.2 % by weight is a well balanced amount.

A conventional clinical study was performed in order to clinically confirm the effects of this cream on the structure of the facial skin with the participation of qualified staff from the dermatological division of the Karolinska hospital. The study included 32 female patients between 40 and 75 years of age having skin types corresponding to I, II, and III according to Fitzpatrick (Validity and Practicality of Sun-Reactive Skin Types I through VI, Archives of Dermatology, Vol 124, p 869-871, June 1988). The patients were administered a randomly coded verum cream for the left or right side of the face and a placebo cream for the other side of the face. The verum cream was composed in accordance with the invention presented here, and the placebo cream had an identical composition except for the absence of lipoic acid. The creams were applied each morning and evening during the test period of twelve weeks. At the beginning of the test, silicon replicas were made beside the canthus at the left and right side of the face respectively, by a trained research nurse. Each patient was photographed by a specialized skin photographer and was assessed regarding eleven different aspects by a physician. The assessment of the physician was repeated at five different occasions, the last occasion taking place at the end of the test period. The making of silicone replicas and the photography were repeated at the end of the test period by the same professionals.

The assessments made by the physician at the end of the test period were compared with the assessments made at the beginning of the test (clinical assessment). The same procedure was used with the photographic comparison (photographic assessment). The silicone replicas at the end of the test period were compared with the corresponding replicas at the beginning of the test period using laser profilometry (laser profilometric measurement). Computer- aided laser profilometry is an objective system for a quantitative analysis of changes in the structure of the skin surface. Finally the participants estimated the result of the treatment using an evaluation form at four occasions during the test period, the last time at the end of this period.

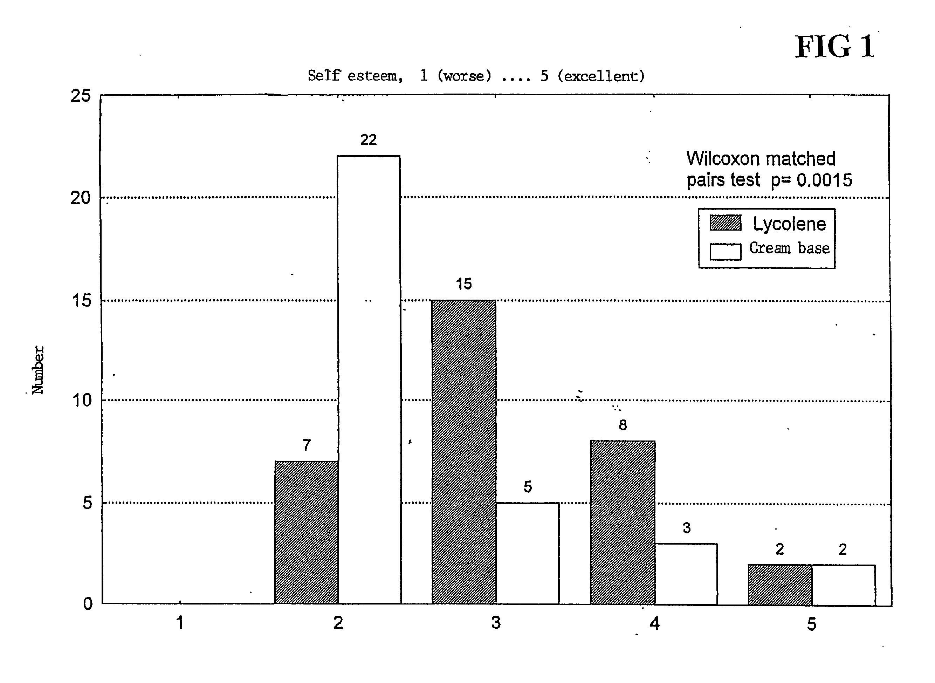

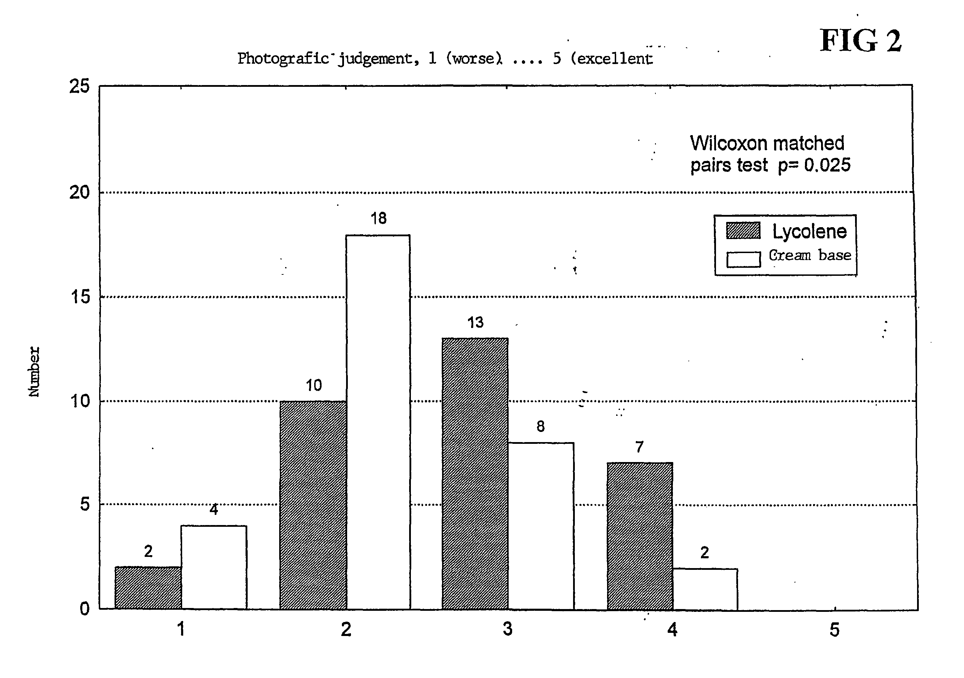

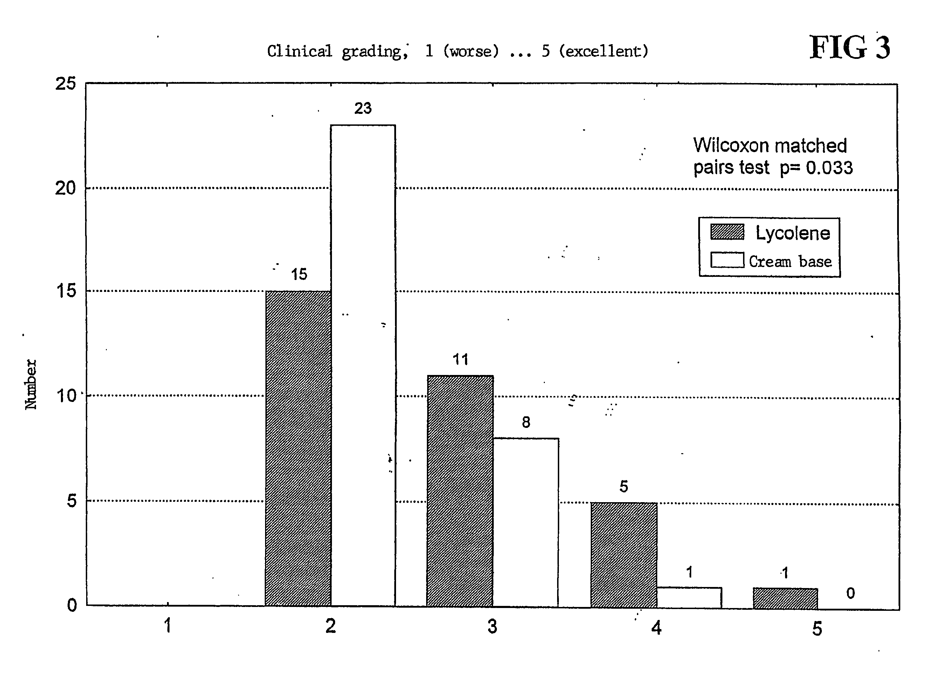

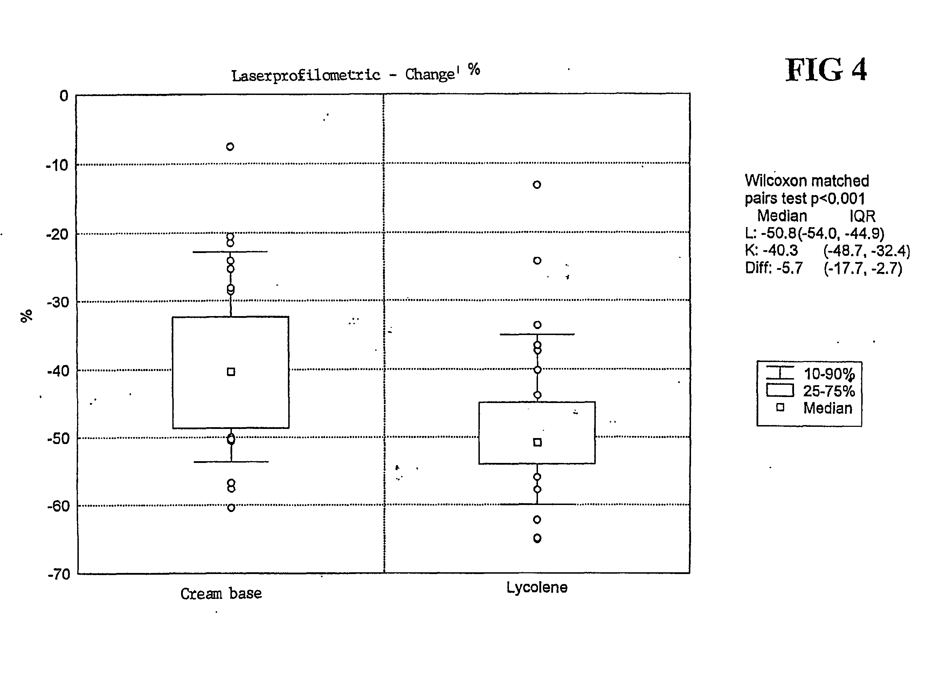

At the clinical and the photographic assessment as well as at the self-appraisal ratings of overall performance the following scale was used: 1 = worse, 2 = no changes identified, 3 = slightly visible/visible improvement, 4 = moderate -obvious improvement, 5 = pronounced - very pronounced improvement. The laserprofilometric measurements show the change calculated as a percentage compared to the initial value. At the evaluation of the results of the test, Wilcoxon's matched pairs test was used. The results are shown in fig. 1 - 4. Fig. 1 (self assessment) p = 0.0015, Fig. 2 (photographic assessment) p = 0.025, Fig. 3 (clinical assessment) p = 0.033, Fig. 4 (laser-profilometric measurement) p < 0.001.

## Claims

1. A facial cream for external treatment of visible sun injuries caused by processes involving the action of free radicals, the active ingredients of which being carried in a cream base, **characterised in that** 0,5 - 7% by weight of D,L-α-lipoic acid, 0,05-0,5% by weight of coenzyme Q-10 and 0,01-3% by weight of acetyl-L-carnitine hydrochloride constitute the active ingredients whereby having a compound synergy.

2. A facial cream according to claim 1, **characterised in that** it comprises 5% by weight of D,L-α-lipoic acid, 0,3 % by weight of coenzyme Q-10 and 0,03% by weight of acetyl-L-carnitine hydrochloride.

3. A facial cream according to claim 1, **characterised in that** it comprises 1% by weight of D,L-α-lipoic acid, 0,3 % by weight of coenzyme Q-10 and 0,03% by weight of acetyl-L-carnitine hydrochloride.

4. A facial cream according to claim 1, where said cream is prepared using a method involving the steps of
- prior to mixing with the remainder of the components of the cream, dissolving the active ingredient D,L-α-lipoic acid in caprylic/capric triglyceride, and
- adding the caprylic/capric triglyceride with the dissolved D,L-α-lipoic acid to a cream base.

## Patentansprüche

1. Gesichtscreme zur äußerlichen Behandlung von sichtbaren Sonnenschädigungen, die durch Reaktionen mit freien Radikalen verursacht werden, wobei die Wirkstoffe in der Creme-Base enthalten sind, **dadurch gekennzeichnet, dass** die Wirkstoffe aus 0,5 - 7 Gew.% D,L-α-Liponsäure, 0,05 - 0,5 Gew.% Coenzym Q10 und 0,01 - 3 Gew.% Acetyl-L-Carnitinhydrochlorid bestehen, wobei eine Synergie zwischen diesen Verbindungen vorliegt.

2. Gesichtscreme gemäß Anspruch 1, **dadurch gekennzeichnet, dass** sie 5 Gew.% D,L-α-Liponsäure, 0,3 Gew.% Coenzym Q10 und 0,03 Gew.% Acetyl-L-Carnitinhydrochlorid umfasst.

3. Gesichtscreme gemäß Anspruch 1, **dadurch gekennzeichnet, dass** sie 1 Gew.% D,L-α-Liponsäure, 0,3 Gew.% Coenzym Q10 und 0,03 Gew.% Acetyl-L-Carnitinhydrochlorid umfasst.

4. Gesichtscreme gemäß Anspruch 1, wobei diese Creme hergestellt wird unter Verwendung eines Verfahrens mit den folgenden Schritten
- vor der Mischung der restlichen Komponenten der Creme, wird der Wirkstoff D,L-α-Liponsäure in Caprylsäure-/Caprinsäure Triglycerid gelöst und
- diese Caprylsäure-/Caprinsäure Triglyceride mit dem gelösten D,L-α-Liponsäure werden zu der Creme-Base hinzugefügt.

## Revendications

1. Crème pour le visage pour le traitement externe des dommages visibles dus au soleil qui ont été causés par des processus impliquant l'action de radicaux libres, les ingrédients actifs de ladite crème étant contenus dans un excipient sous forme de crème, **caractérisée en ce que** lesdits ingrédients actifs sont composés de 0,5-7% en poids de D,L acide α-lipoïdique, 0,05-0,5% en poids de coenzyme Q-10 et 0,01-3% en poids d'hydrochlorure d'acétate de L-carnitine, moyennant quoi ils agissent en synergie.

2. Crème pour le visage selon la revendication 1, **caractérisée en ce qu'**elle contient 5% en poids de D,L acide α-lipoïdique, 0,3% en poids de coenzyme Q-10 et 0,03% en poids d'hydrochlorure d'acétate de L-carnitine.

3. Crème pour le visage selon la revendication 1, **caractérisée en ce qu'**elle contient 1% en poids de D,L acide α-lipoïdique, 0,3% en poids de coenzyme Q-10 et 0,03% en poids d'hydrochlorure d'acétate de L-carnitine.

4. Crème pour le visage selon la revendication 1, où elle est préparée selon un procédé comprenant les étapes de :
- dissolution du D,L acide α-lipoïdique dans un triglycéride octylique/caprique avant le mélange avec les autres ingrédients de ladite crème ; et
- ajout du D,L acide α-lipoïdique dissout dans le triglycéride octylique/caprique dans un excipient sous forme de crème.
